(19) Europäisches Patentamt / European Patent Office / Office européen des brevets

(11) EP 2 229 193 B1

(12) # EUROPÄISCHE PATENTSCHRIFT

(45) Veröffentlichungstag und Bekanntmachung des Hinweises auf die Patenterteilung:
**28.11.2012 Patentblatt 2012/48**

(21) Anmeldenummer: **08864705.2**

(22) Anmeldetag: **16.12.2008**

(51) Int Cl.:
***A61L 31/16*** *(2006.01)* ***A61L 15/46*** *(2006.01)*

(86) Internationale Anmeldenummer:
**PCT/EP2008/010679**

(87) Internationale Veröffentlichungsnummer:
**WO 2009/080239 (02.07.2009 Gazette 2009/27)**

(54) **MEDIZINISCHER ARTIKEL MIT ANTIMIKROBIELLER AUSRÜSTUNG**

MEDICAL ARTICLE HAVING AN ANTIMICROBIAL FINISH

ARTICLE MÉDICAL PRÉSENTANT UN APPRÊT ANTIMICROBIEN

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **22.12.2007 DE 102007062372**

(43) Veröffentlichungstag der Anmeldung:
**22.09.2010 Patentblatt 2010/38**

(73) Patentinhaber: **Paul Hartmann Aktiengesellschaft**
**89522 Heidenheim (DE)**

(72) Erfinder: **HIETKAMP, Peter**
**89518 Heidenheim (DE)**

(56) Entgegenhaltungen:
**WO-A1-2006/066752**
**DE-A1- 19 729 025**
**DE-A1-102004 011 293**
**US-A1- 2004 082 925**

EP 2 229 193 B1

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

## Beschreibung

Die Erfindung betrifft medizinische oder medizintechnische Artikel nämlich Binden, Gesichtsmasken, Operationsabdeckungen, Patientenabdeckungen oder Operationsmäntel mit einer antimikrobiellen Ausrüstung.

Antimikrobiell ausgerüstete Gegenstände sind in der Medizin und in der Medizintechnik lange Zeit bekannt. Hierbei soll die antimikrobielle Ausrüstung sowohl den Gegenstand bzw. das Material aus dem der Gegenstand besteht vor dem Befall durch Mikroorganismen schützen oder zumindest die Vermehrung von Mikroorganismen in oder auf dem Gegenstand unterdrücken, als auch die unmittelbare Umgebung des Artikels vor dem Befall durch Mikroorganismen bewahren oder zumindest die Vermehrung von Mikroorganismen in dieser Umgebung unterdrücken. Dabei werden unterschiedlichste antimikrobielle Wirkstoffe (Mikrobizide) zum Einsatz gebracht. Die eingesetzten Mikrobizide sind Stoffe unterschiedlicher chemischer Zusammensetzung, die Mikroorganismen wie beispielsweise Viren, Bakterien, Pilze oder Algen abtöten können. Daher werden Mikrobizide u.a. auch nach ihrer Wirksamkeit unterschieden in Antibiotika, Konservierungsmittel, Desinfektionsmittel, Schleimbekämpfungsmittel und Anstrichfungizide oder auch nach ihrer spezifischen Wirkung gegen verschiedene Mikroorganismengruppen unterschieden in Algizide, Bakterizide, Fungizide und Viruzide. Wirkstoffe, die die Vegetation von Mikroorganismen nur hemmen, ohne sie abzutöten, bezeichnet man auch als Mikrobistatika wie beispielsweise Bakteriostatika oder Virostatika.

Über die chemische Struktur und die Eigenschaften der bekannten Mikrobizide besitzt man meist genaue Kenntnisse. Dagegen ist bezüglich ihrer Wirkungsorte in den Zellen und über die in den Zellen auf molekularer Ebene ablaufenden Wirkungsmechanismen wenig bekannt. Nichtsdestotrotz kann man eine große Anzahl von Mikrobiziden nach ihrem Wirkungsmechanismus charakterisieren als membranaktive oder elektrophilaktive Wirkstoffe. Eine eindeutige Zuordnung ist jedoch nicht immer möglich.

Zu den membranaktiven Mikrobiziden zählen beispielsweise: Alkohole, Säuren, Phenole, Salicylanilide, Carbanilide, Dibenzamidine, Wirkstoffe mit kationogenem Charakter, wie quartäre Ammonium-Verbindungen, Biguanide und Azol-Fungizide. Den membranaktiven Wirkstoffen ist gemeinsam, dass sie die Zellmembran adsorptiv belegen können. Vor allem in Gegenwart nicht letaler Mikrobizidkonzentrationen ist dies zunächst ein reversibler Vorgang, der jedoch zu Veränderungen an der äußeren Membran und der Zellwand führt. Die äußeren Barrieren verlieren ihre Integrität, wodurch der Zugang zur Cytoplasmamembran für die Mikrobizidmoleküle ermöglicht wird und somit die letalen Effekte ausgelöst werden können.

Aus der DE 10 2004 011 293 A1 sind Medizinprodukte bekannt, welche im Inneren des Körpers als Nahtmaterialien, Herniennetze, Harninkontinenznetze, Gefässprothesen, orthopädische Implantate oder außen am Körper als Wundauflagen einsetzbar sind und im Bereich der Oberfläche mit Polyhexamenthylenbiguanid (PHMB) als unspezifisch antimikrobiell wirksame Komponente ausgerüstet sind.

Aufgabe der vorliegenden Erfindung ist es nun, außerhalb der Körpers einsetzbare, medizinische und medizintechnische Produkte mit unspezifisch aber dennoch hoch wirksamen antimikrobiellen Ausrüstungen zur Verfügung zu stellen, die die Besiedelung durch pathogene Keime auf den Produkten und in der näheren Umgebung der Produkte effektiv hemmen oder verhindern. Hierbei soll insbesondere sichergestellt sein, dass weder der Anwender noch ein Patient durch den Umgang mit dem Produkt in seiner Gesundheit beeinträchtigt wird. Durch diese Ausrüstung soll weithin sichergestellt sein, dass auch das Wachstum von denjenigen Keimen effektiv gehemmt oder gehindert wird, die gegen bereits eingesetzte antimikrobiell wirkende Mittel Resistenzen aufgebaut haben.

Gelöst wird die Aufgabe durch einen medizinischen oder medizintechnischen Artikel gemäß Anspruch 1 umfassend eine antimikrobielle Zubereitung und ein textiles Trägermaterial mit einer ersten oberflächenbildenden Seite und einer zweiten oberflächenbildenden Seite, wobei die Zubereitung auf der ersten Seite des Trägermaterials aufgebracht ist, und wobei die Zubereitung Polyhexamethylenguanidin oder ein Salz hiervon als antimikrobielles Mittel und weitere Hilfs- oder Zusatzstoffe umfasst.

Dabei weist Polyhexamethylenguanidine gemäß der vorliegenden Erfindung die folgende chemische Struktur gemäß Formel (I) auf:

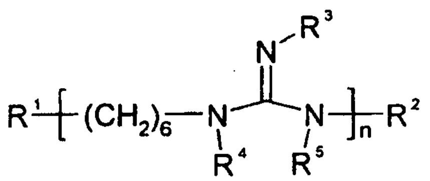

(I)

wobei gilt:

n = 10 bis 80

$R^1$ = H, OH, $NH_2$ oder NH-C(=NH)-$NH_2$;

$R^2$, $R^3$, $R^4$, $R^5$ = gleichzeitig oder unabhängig voneinander H, OH, $(CH_2)_6$-$NH_2$ oder $(CH_2)_6$-OH.

Bevorzugt bedeutet gemäß Formel (I) gleichzeitig oder unabhängig voneinander:

n = 10 bis 80
$R^1$ = H, $NH_2$ oder NH-C(=NH)-$NH_2$;
$R^2$ = H oder $(CH_2)_6$-$NH_2$;
$R^3$, $R^4$, $R^5$= H.

Weiterhin bevorzugt kann n = 20 bis 80, insbesondere n = 30 bis 80, insbesondere n = 30 bis 70 oder besonders bevorzugt n = 30 bis 60 bedeuten. Es kann jedoch auch vorgesehen sein, dass n = 35 bis 80, insbesondere n = 35 bis 70, insbesondere n = 35 bis 60, insbesondere
n = 40 bis 80 oder besonders bevorzugt n = 40 bis 70 bedeutet.

Weiterhin kann das Polyhexamethylenguanidin gemäß der vorliegenden Erfindung auch als Kation insbesondere als Polykation gemäß der folgenden Formel (II) vorliegen:

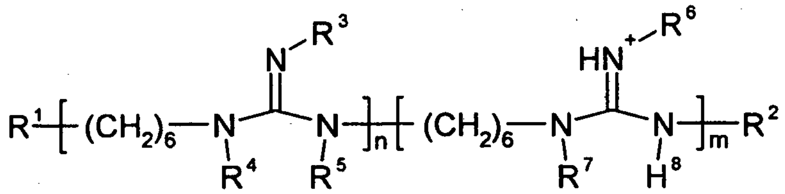

$m/p \; X^{p-}$ (II)

wobei gilt: m = 1 bis 80 und n = 0 bis 79, wobei $10 \leq n + m \leq 80$ gilt;
$R^1$ = H, OH, $NH_2$, $NH_2^+$, NH-C(=NH)-$NH_2$ oder NH-C(=$NH_2^+$)-$NH_2$;
$R^2$, $R^3$, $R^4$, $R^5$ = gleichzeitig oder unabhängig voneinander H, OH, $(CH_2)_6$-$NH_2$, $(CH_2)_6$-OH oder $(CH_2)_6$-$NH_2^+$;
$R^6$, $R^7$, $R^8$= gleichzeitig oder unabhängig voneinander H, $(CH_2)_6$-$NH_2$, $(CH_2)_6$-OH oder $(CH_2)_6$-$NH_2^+$;
p = 1, 2 oder 3; und
X = ein organisches oder anorganisches Anion, das gemäß

p = 1 ein einwertiges organisches oder anorganisches Anion,
p = 2 ein zweiwertiges organisches oder anorganisches Anion oder
p = 3 ein dreiwertiges organisches oder anorganisches Anion ist.

In einer bevorzugten Ausführung der Erfindung bedeutet in Formel (II) gleichzeitig oder unabhängig voneinander:

m = 1 bis 80 und n = 0 bis 79, wobei $10 \leq n + m \leq 80$ gilt;
$R^1$ = H, $NH_2$, $NH_2^+$, NH-C(=NH)-$NH_2$ oder NH-C(=$NH_2^+$)-$NH_2$;
$R^2$ , $R^3$, $R^4$, $R^5$ = gleichzeitig oder unabhängig voneinander H, $(CH_2)_6$-$NH_2$ oder $(CH_2)_6$-$NH_2^+$;
$R^6$, $R^7$, $R^8$ = gleichzeitig oder unabhängig voneinander H, $(CH_2)_6$-$NH_2$, oder $(CH_2)_6$-$NH_2^+$;
p = 1, 2 oder 3; und
X = ein organisches oder anorganisches Anion, das gemäß

p = 1 ein einwertiges organisches oder anorganisches Anion,
p = 2 ein zweiwertiges organisches oder anorganisches Anion oder
p = 3 ein dreiwertiges organisches oder anorganisches Anion ist.

In einer weiteren bevorzugten Ausführung der Erfindung bedeutet in Formel (II) gleichzeitig oder unabhängig voneinander:

m = 1 bis 80 und n = 0 bis 79, wobei $10 \leq n + m \leq 80$ gilt;
$R^1$ = H, $NH_2^+$, NH-C(=$NH_2^+$)-$NH_2$, $NH_2$ oder NH-C(=$NH_2$)-$NH_2$;
$R^2$ = H, $(CH_2)_6$-$NH_2$ oder $(CH_2)_6$-$NH_2$;
$R^3$, $R^4$, $R^5$, $R^6$, $R^7$, $R^8$= H;
p = 1, 2 oder 3; und
X = ein organisches oder anorganisches Anion, das gemäß

p = 1 ein einwertiges organisches oder anorganisches Anion,
p = 2 ein zweiwertiges organisches oder anorganisches Anion oder
p = 3 ein dreiwertiges organisches oder anorganisches Anion ist.

Weiterhin bevorzugt kann gemäß Formel (II) der vorliegenden Erfindung insbesondere $20 \leq n + m \leq 80$, insbesondere $30 \leq n + m \leq 80$, insbesondere $30 \leq n + m \leq 70$ und besonders bevorzugt $30 \leq n + m \leq 60$ bedeuten. Es kann jedoch auch vorgesehen sein, dass $35 \leq n + m \leq 80$, insbesondere $35 \leq n + m \leq 70$, insbesondere $30 \leq n + m \leq 60$, insbesondere $40 \leq n + m \leq 80$ und besonders bevorzugt $40 \leq n + m \leq 70$ bedeutet. Gleichzeitig oder unabhängig hiervon kann gemäß Formel (II) der vorliegenden Erfindung insbesondere m = 10 bis 80, insbesondere m = 20 bis 80, insbesondere m = 30 bis 80, insbesondere m = 30 bis 70 oder besonders bevorzugt m = 30 bis 60 und n = 0 bis 79 bedeuten. Es kann jedoch auch vorgesehen sein, dass m = 5 bis 80, insbesondere m = 15 bis 80, insbesondere m = 25 bis 80, insbesondere m = 35 bis 80, insbesondere m = 40 bis 80, insbesondere m = 35 bis 70, insbesondere m = 35 bis 60, insbesondere m = 40 bis 80, insbesondere m = 40 bis 70 und n = 0 bis 60 bedeutet.

Damit kann weiterhin bevorzugt das Polyhexamethylenguanidin gemäß der vorliegenden Erfindung auch als Salz vorliegen. Falls das Polyhexamethylenguanidin als Salz vorliegt, so liegt das Grundpolymer als Kation oder Polykation vor, dessen positive Ladungen durch einwertige, zweiwertige oder dreiwertige anorganische oder organische Anionen ausgeglichen sind. Insbesondere sind als Anionen Chlorid, Bromid, Nitrat, Phosphat, Hydrogenphosphat, Dihydrogenphosphat, Citrat, Hydrogencitrat, Dihydrogencitrat, Sulfat, Hydrogensulfat, Carbonat, Hydrogencarbonat, Acetat, Gluconat, Lactat oder Mischungen hiervon geeignet. Besonders bevorzugt ist Chlorid, Phosphat und Hydrogenphosphat.

Ganz besonders bevorzugt umfasst die Zubereitung Polyhexamethylenguanidiniumhydrochlorid (CAS 57 028-96-3).

Überraschender Weise hat sich gezeigt, dass Polyhexamethylenguanidin oder ein Salz hiervon, insbesondere Polyhexamethylenguanidiniumhydrochlorid ein breites antimikrobielles Wirkspektrum aufweist. Polyhexamethylenguanidin oder ein Salz hiervon ist beispielsweise wirksam gegen Bakterien wie Echerichia coli, Pseudomonas aeruginosa, Staphylococcus aureus, Salmonella typhimurium, Salmonella gallinarium, Mycobacterium tuberculosis, Clostridium difficile, Coryme bacterium diphteriae, Shigella sonnei, Shigella flexneri, Proteus vulgaris, Pasteurella gallinarium oder Listeria monocitogenes. Zudem ist es wirksam gegen zahlreiche Pilze und Vieren. Weiterhin überraschend hat sich gezeigt, dass auch Polyhexamethylenguanidin oder ein Salz hiervon, insbesondere Polyhexamethylenguanidiniumhydrochlorid auch antibakteriell wirksam ist gegen Bakterien, die bereits Resistenzen aufgebaut haben, wie beispielsweise Staphylococcus aureus (MRSA).

Aufgrund des polymeren Charakters ist Polyhexamethylenguanidin oder dessen Salze gegenüber bekannten niedermolekularen antimikrobiellen Verbindungen wie beispielsweise niedermolekulare quaternäre Stickstoffverbindungen z.B. Chlorhexidin oder Benzalkoniumchlorid wenig toxisch und weist eine hohe Stabilität gegen Ultraviolette Strahlung auf. Aufgrund des polymeren Charakters kann Polyhexamethylenguanidin oder dessen Salze nicht über die Haut in den Körper eines Menschen gelangen. Somit kann ein mit Polyhexamethylenguanidin oder dessen Salz ausgerüsteter Gegenstand direkt auf der Haut getragen werden.

Überraschender Weise hat sich zudem gezeigt, dass Polyhexamethylenguanidin auf einem textilen Trägermaterial über einen langen Zeitraum antibakteriell wirkt. Damit ist gemäß der vorliegenden Erfindung auch ein medizinischer oder medizintechnischer Artikel ein bevorzugter Gegenstand, der ab dem erstmaligen Gebrauch mindestens 7 Tage, vorzugsweise mindestens 14 Tage, antimikrobiell wirksam ist.

Hiervon losgelöst oder im Zusammenhang stehend ist auch ein medizinischer oder medizintechnischer Artikel ein bevorzugter Gegenstand der vorliegenden Erfindung, dessen Gehalt an Polyhexamethylenguanidin oder dessen Salz bezogen auf die Oberfläche des Artikels 0,0001 bis 100 mg/ $cm^2$ beträgt. Insbesondere weist der Artikel einen Gehalt von 0,1 bis 800 $\mu$g/ $cm^2$, insbesondere 0,1 bis 400 $\mu$g/ $cm^2$, insbesondere 0,1 bis 150 $\mu$g/ $cm^2$ und ganz besonders bevorzugt 0,1 bis 100 $\mu$g/ $cm^2$uf.

Gemäß der vorliegenden Erfindung ist dabei vorgesehen, dass eine Zubereitung umfassend Polyhexamethylenguanidin oder dessen Salz auf eine erste oberflächenbildenden Seite eines textilen Trägermaterials aufgebracht ist. Als textiles Trägermaterial können dabei insbesondere Faservliese, so genannte Nonwoven, oder auch echte textile Trägermaterialien wie Gewebe, Gewirke oder Gestricke zum Einsatz gebracht werden. Als Faservliese eignen sich insbesondere Kardenvliese, Stapelfaservliese, Spinnvliese (im Spinnverfahren hergestelltes Faservlies), Meltblownvliese (im Meltblown-Verfahren hergestellte Faservliese) oder auch Laminate aus mindestens zwei Faservliesen. Es können jedoch auch Laminate aus einer Folie und mindestens einem Faservlies eingesetzt werden, wobei weiterhin bevorzugt diese Faservliese selbst aus einem Laminat aus mindestens zwei Faservliesen besteht. Besonders bevorzugt sind Stapelfaservliese oder Laminate aus mindestens zwei Faservliesen oder Laminate aus mindestens drei Faservliesen, wie beispielsweise ein Spunbond-Meltblown-Faservlies (SM-Nonwoven), ein Spunbond-Meltblown-Spunbond-Faservlies (SMS-Nonwoven) oder ein Spunbond-Meltblown-Meltblown-Spunbond-Faservlies (SMMS-Nonwoven) .

Insbesondere ist als Stapelfaservlies ein Faservlies einsetzbar, das aus Stapelfasern hergestellt ist und das

mittels Wasserstrahlen und/ oder thermischer Verfestigung stabilisiert oder verfestigt ist. Hierbei sind weiterhin bevorzugt solche Stapelfasern einzusetzen, die eine Faserlänge von 3 bis 60 mm aufweisen. Diese Stapelfasern können insbesondere aus organischen Fasern bestehen, die synthetische und/oder natürliche Polymere umfassen. Ganz besonders bevorzugt ist ein Fasermaterial, das aus Viskosefasern, Cellulosefasern, Alginatfasern, Acetatfasern, Polyersterfasern, Polyamidfasern, Polyethylenfasern, Polypropylenfasern oder Kombinationen hiervon besteht. Weiterhin bevorzugt hiervon ist ein Fasermaterial, das aus Viskosefasern und/ oder Cellulosefasern besteht.

Für die Herstellung der erfindungsgemäßen medizinischen oder medizintechnischen Artikel haben sich Stapelfasern einer Länge von 3 bis 60 mm bewährt. Als Stapelfasern werden Fasern einer definierten Länge verstanden, welche Chemiefasern, also aus natürlichen oder synthetischen Polymeren industriell hergestellte Fasern, und natürliche Fasern sein können. Wenn Chemiefasern als Stapelfasern verwendet werden, so können diese in vorteilhafter Weiterbildung auch Längen von 15 bis 40 mm, insbesondere von 15 bis 25 mm aufweisen. Die Länge von natürlichen Stapelfasern, wie beispielsweise Baumwollfasern betragen vorteilhafterweise 9 bis 15 mm, insbesondere etwa 12 mm.

Die vorstehend erwähnten Chemiefasern als Stapelfasern können in vorteilhafter Weise Mikrostapelfasern umfassen oder Mikrostapelfasern sein. Der Begriff Mikrostapelfasern bedeutet hierbei unabhängig von der Faserlänge, dass diese Fasern eine Faserstärke von kleiner als 1 dtex haben.

Mikrostapelfasern können in vorteilhafter Weise Polyester(PES)-Fasern oder Viskosefasern sein (Viskosefasern bestehen zwar aus natürlichen Zellulosemolekülen, die aber synthetisch zur Bildung von Fasern verarbeitet werden).

Im Unterschied zu Stapelfaservliesen bestehen Spinnvliese im Wesentlichen aus Endlosfilamenten. Zur Herstellung dieser Filamente, aus dem ein Spinnvlies besteht, wird ein Prozess verwendet, der dem Prinzip der herkömmlichen Faserherstellung entspricht. Im Schmelzspinnverfahren wird dazu ein Kunststoffgranulat aufgeschmolzen und einer Spinndüse zugeführt. Die daraus austretenden Filamente werden aerodynamisch oder mechanisch abgezogen, verstreckt und direkt der Faserlegung zur Herstellung des Spinnvlieses zugeführt. Im letzten Schritt der Vliesherstellung kann nun dieses Spinnvlies einem Verfestigungsschritt zugefügt werden. Diese Spinnvliese können insbesondere als Oberflächenvliese verwendet werden.

Zur Herstellung eines Faservlieses, insbesondere eines Stapelfaservlieses oder eines Spinnvlieses oder eines Meltblownvlieses als Trägermaterial gemäß der der vorliegenden Erfindung können nun grundsätzlich verschiedene Verfahren angewendet werden. Einerseits ist es möglich, dass das Polyhexamethylenguanidin oder ein Salz hiervon, insbesondere Polyhexamethylenguanidiniumchlorid direkt der Spinnmasse zur Herstellung von Fasern oder Filamenten zugesetzt wird. Andererseits kann das

Polyhexamethylenguanidin oder ein Salz hiervon, insbesondere

Polyhexamethylenguanidiniumchlorid nach der Filament- oder Faserbildung oder nach der Vlieslegung aufgebracht, insbesondere gesprüht werden. Es ist auch möglich das verfestigte Faservlies, insbesondere ein verfestigtes Stapelfaservlies oder verfestigte Spinnvlies in einem nachgeschaltetem Arbeitsschritt mit einer Lösung umfassend Polyhexamethylenguanidin oder ein Salz hiervon, insbesondere Polyhexamethylenguanidiniumchlorid zu beaufschlagen, insbesondere durch Besprühen oder Tauchen:

Weiterhin sind solche Trägermaterialien bevorzugt, die ein Flächengewicht von 10 bis 250 g/ $m^2$, insbesondere 15 bis 150 g/ $m^2$ und ganz besonders bevorzugt 20 bis 100 g/ $m^2$ aufweisen. Werden Faservliese als Trägermaterialien eingesetzt, so können insbesondere solche Faservliese verwendet werden, die ein Flächengewicht von 10 bis 100 g/$m^2$, insbesondere von 10 bis 80 g/$m^2$ und ganz besonders bevorzugt von 10 bis 70 g/$m^2$ aufweisen. Werden dagegen Gewebe, Gewirke oder Gestricke als Trägermaterialien eingesetzt, so weisen diese ein Flächengewicht von 50 bis 250 g/$m^2$, insbesondere von 70 bis 250 g/$m^2$ und ganz besonders bevorzugt von 80 bis 200 g/$m^2$ auf.

Des Weiteren sind solche Trägermaterialien, insbesondere Faservliese bevorzugt, die eine Dichte von 0,01 bis 0,20 g/$cm^3$ insbesondere 0,05 bis 0,20 g/$cm^3$ und ganz besonders bevorzugt 0,07 bis 0,15 g/$cm^3$ aufweist (Dicke gemessen bei einem Prüfdruck von 10 cN/$cm^2$).

In einer weiteren Ausführung der vorliegenden Erfindung kann vorgesehen sein, dass das Trägermaterial derart in dem medizinischen oder medizintechnischen Artikel angeordnet ist, dass die mit der Zubereitung beaufschlagte erste Seite des Trägermaterials zum Körper des behandelnden Arztes oder des Patienten zeigt somit der Haut des behandelnden Arztes oder des Patienten direkt benachbart ist oder auf der Haut behandelnden Arztes oder des Patienten aufgelegt werden kann. Es kann jedoch auch eine Abstandsschicht vorgesehen sein, die die erste Seite des Trägermaterials von der Haut behandelnden Arztes oder des Patienten von der Haut behandelnden Arztes oder des Patienten beabstandet, derart, dass die mit der Zubereitung beaufschlagte Seite des Trägermaterials zwischen der zweiten oberflächenbildenden Seite des Trägermaterials und der Abstandsschicht eingeschlossen ist. Diese Abstandsschicht wiederum kann aus einem von dem Trägermaterial verschiedenem oder gleichem Material bestehen. Insbesondere ist als Abstandschicht eine textile Abstandsschicht und weiterhin bevorzugt ein Faservlies, ein Gewirk, ein Gewebe oder ein Gestrick vorgesehen. Damit ist gemäß der vorliegenden Erfindung auch ein medizinischer oder medizintechnischer Artikel eine bevorzugte Ausführungsform, der mindestens eine Abstandsschicht mit einer ersten Seite und einer zweiten Seite aufweist, wobei die erste Seite der Abstandsschicht der ersten Seite des Trägermaterials benachbart ist.

Die Zubereitung umfasst als Hilfs- oder Zusatzstoff ein Hautpflegemittel, ein Tensid, ein Hydrophobisierungsmittel, ein Hydrophilisierungsmittel und/oder ein Avivagemittel. Hierbei ist insbesondere vorgesehen, dass eine Menge an Hilfs- oder Zusatzstoff bezogen auf die Oberfläche des Artikels 0,0001 bis 150 mg/ $cm^2$ beträgt. Insbesondere weist der Artikel einen Gehalt von 0,1 bis 900 $\mu g/cm^2$, insbesondere 0,1 bis 500 $\mu g/cm^2$, insbesondere 0,1 bis 300 $\mu g/cm^2$ und ganz besonders bevorzugt 0,1 bis 150 $\mu g/cm^2$ auf.

Insbesondere weist die Zubereitung kein Wasser auf. Somit ist der erfindungsgemäße Artikel insbesondere bis auf einen durch vorhandene Luftfeuchtigkeit verursachten Restwassergehalt als wasserfrei zu bezeichnen.

Vorzugsweise umfasst die Zubereitung als Hautflegemittel insbesondere einen Pflanzenextrakt, wobei der Pflanzenextrakt beispielsweise durch Extraktion der gesamten Pflanze, aber auch ausschließlich durch Extraktion aus Blüten, Blättern, Samen, Wurzeln und/oder anderen Pflanzenteilen, hergestellt werden kann. Erfindungsgemäß sind vor allem die Extrakte aus speziellen Pflanzen wie Grünem Tee, Hamamelis, Kamille, Stiefmütterchen, Aloe Vera, Rosskastanie, Salbei, Weidenrinde, Chrysanthemen, Eichenrinde, Brennnessel, Hopfen, Klettenwurzel, Schachtelhalm, Weissdorn, Lindenblüten, Mandeln, Fichtennadeln, Sandelholz, Wacholder, Kokosnuss, Kiwi, Guave, Limette, Mango, Aprikose, Weizen, Melone, Orange, Grapefruit, Avocado, Rosmarin, Birke, Buchensprossen, Wiesenschaumkraut, Schafgarbe, Quendel, Thymian, Melisse, Eibisch, Veilchen, Blättern der Schwarzen Johannisbeere, Huflattich, Fünffingerkraut, Ginseng, Ingwerwurzel und Süßkartoffel als Pflanzenextrakt bevorzugt.

Insbesondere umfasst die Zubereitung einen Pflanzenextrakt aus Grünem Tee, Hamamelis, Ringelblume oder Aloe Vera. Die erfindungsgemäßen Zubereitungen können auch Mischungen aus mehreren, insbesondere aus zwei, verschiedenen Pflanzenextrakten enthalten. In einer besonders bevorzugten Ausführungsform ist in der erfindungsgemäßen Zubereitung Aloe Vera enthalten.

Vorzugsweise umfasst die Zubereitung als Tensid insbesondere ein nichtionisches Tensid, ein kationisches Tensid, ein anionisches Tensid oder ein amphoteres Tensid.

Als nichtionische Tenside werden vorzugsweise alkoxylierte, vorteilhafterweise ethoxylierte, insbesondere primäre Alkohole mit vorzugsweise 8 bis 18 C-Atomen und durchschnittlich 1 bis 12 Mol Ethylenoxid (EO) pro Mol Alkohol eingesetzt, in denen der Alkoholrest linear oder bevorzugt in 2-Stellung methylverzweigt sein kann bzw. lineare und methylverzweigte Reste im Gemisch enthalten kann, so wie sie üblicherweise in Oxoalkoholresten vorliegen. Insbesondere sind jedoch Alkoholethoxylate mit linearen Resten aus Alkoholen nativen Ursprungs mit 12 bis 18 C-Atomen, z.B. aus Kokos-, Palm-, Talgfett- oder Oleylalkohol, und durchschnittlich 2 bis 8 EO pro Mol Alkohol bevorzugt. Zu den bevorzugten ethoxylierten Alkoholen gehören beispielsweise C12-14-Alkohole mit 3 EO bis 7 EO, C9-11-Alkohol mit 7 EO, C13-15-Alkohole mit 3 EO, 5 EO, 7 EO oder 8 EO, C12-18-Alkohole mit 3 EO, 5 EO oder 7 EO und Mischungen aus diesen, wie Mischungen aus C12-14-Alkohol mit 3 EO und C12-18-Alkohol mit 7 EO. Die angegebenen Ethoxylierungsgrade stellen statistische Mittelwerte dar, die für ein spezielles Produkt eine ganze oder eine gebrochene Zahl sein können. Zusätzlich zu diesen nichtionischen Tensiden können auch Fettalkohole mit mehr als 12 EO eingesetzt werden. Beispiele hierfür sind Talgfettalkohol mit 14 EO, 25 EO, 30 EO oder 40 EO. Auch nichtionische Tenside, die EO- und PO-Gruppen zusammen im Molekül enthalten, sind erfindungsgemäß einsetzbar. Hierbei können Blockcopolymere mit EO-PO-Blockeinheiten bzw. PO-EO-Blockeinheiten eingesetzt werden, aber auch EO-PO-EO-Copolymere bzw. PO-EO-PO-Copolymere. Selbstverständlich sind auch gemischt alkoxylierte Niotenside einsetzbar, in denen EO- und PO-Einheiten nicht blockweise sondern statistisch verteilt sind. Solche Produkte sind durch gleichzeitige Einwirkung von Ethylenoxid (EO) und Propylenoxid (PO) auf Fettalkohole erhältlich.

Beispiele für Fettalkoholpolyethylenglycolether sind solche mit der Formel (III)

$$R^1O\text{-}(CH_2CH_2O)_n\text{-}H \qquad \text{(III)}$$

wobei für gilt:

- n = 1 bis 5;
- $R^1$ = eine linearer oder verzweigter Alkyl- und/oder Alkenylrest mit 6 bis 22, vorzugsweise 12 bis 18 Kohlenstoffatomen.

Als kationische Tenside können insbesondere quartäre Ammoniumverbindungen, kationische Polymere und Emulgatoren, wie in Mitteln zur Textilavivage eingesetzt werden. Geeignete Beispiele für quartäre Ammoniumverbindungen sind Stoffe der Formeln (IV) und (V):

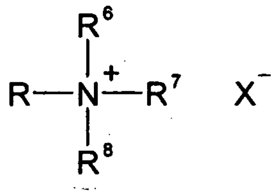

(IV)

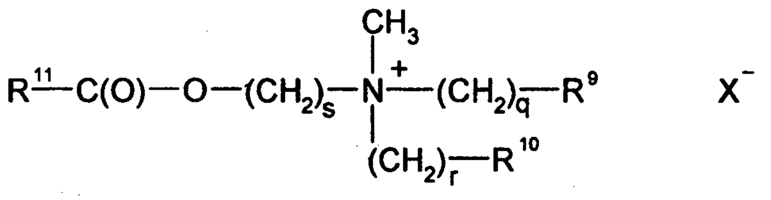

(V)

wobei in Formel (IV) gilt:

R, $R^6$ = ein acyclischer Alkylrest mit 12 bis 24 Kohlenstoffatomen;
$R^7$ = ein gesättigter C1-C4 Alkyl- oder Hydroxyalkylrest;
$R^8$ = entweder gleich R, Ra oder Rb ist oder für einen aromatischen Rest steht;
$X^-$ = ein Halogenid-, Methosulfat-, Methophosphat- oder Phosphatanion sowie Mischungen hiervon.

Verbindungen der Formel (V) sind sogenannte Esterquats. Hierfür gilt:

$R^9$ = H, OH oder O(CO)-$R^{12}$, wobei $R^{12}$ unabhängig von $R^{11}$ und $R^{13}$ für einen aliphatischen Alkylrest mit 12 bis 22 Kohlenstoffatomen oder für einen aliphatischen Hydroxyalkylrest mit 12 bis 22 Kohlenstoffatomen oder für einen aliphatischen Alkenylrest mit 12 bis 22 Kohlenstoffatomen mit 1, 2 oder 3 Doppelbindungen steht;

$R^{10}$ = H, OH oder O(CO)-$R^{13}$, wobei $R^{13}$ unabhängig von $R^{12}$ und $R^{11}$ für einen aliphatischen Alkylrest mit 12 bis 22 Kohlenstoffatomen oder für einen aliphatischen Hydroxyalkylrest mit 12 bis 22 Kohlenstoffatomen oder für einen aliphatischen Alkenylrest mit 12 bis 22 Kohlenstoffatomen mit 1, 2 oder 3 Doppelbindungen steht;

$R^{11}$ = unabhängig von $R^{12}$ und $R^{13}$ ein aliphatischer Alkylrest mit 12 bis 22 Kohlenstoffatomen oder ein aliphatischen Hydroxyalkylrest mit 12 bis 22 Kohlenstoffatomen oder ein aliphatischer Alkenylrest mit 12 bis 22 Kohlenstoffatomen mit 1, 2 oder 3 Doppelbindungen;

q, r, s = jeweils unabhängig voneinander 0, 1, 2, 3, 4 oder 5, wobei jedoch $q + r + s \geq 1$ gilt;

$X^-$ = Halogenid-, Methosulfat-, Methophosphat- oder Phosphation sowie Mischungen hiervon.

Für bevorzugte Verbindungen gemäß Formel V gilt:

$R^9$ = O(CO)-$R^{12}$, wobei $R^{12}$ unabhängig von $R^{11}$ und $R^{13}$ für einen aliphatischen Alkylrest mit 16 bis 18 Kohlenstoffatomen oder für einen aliphatischen Alkenylrest mit 16 bis 18 Kohlenstoffatomen mit 1, 2 oder 3 Doppelbindungen steht;

$R^{10}$ = O(CO)-$R^{13}$, wobei $R^{13}$ unabhängig von $R^{11}$ und $R^{13}$ für einen aliphatischen Alkylrest mit 16 bis 18 Kohlenstoffatomen oder für einen aliphatischen Alkenylrest mit 16 bis 18 Kohlenstoffatomen mit 1, 2 oder 3 Doppelbindungen steht;

$R^{11}$ = unabhängig von $R^{12}$ und $R^{13}$ ein aliphatischer Alkylrest mit 16 bis 18 Kohlenstoffatomen oder ein aliphatischer Alkenylrest mit 16 bis 18 Kohlenstoffatomen mit 1, 2 oder 3 Doppelbindungen;

q, r, s = jeweils unabhängig voneinander 0, 1, 2 oder 3, wobei jedoch $q + r + s \geq 1$ gilt;

X = Halogenid-, Methosulfat-, Methophosphat- oder Phosphation sowie Mischungen hiervon.

Beispiele für kationische Verbindungen der Formel (IV) sind insbesondere Didecyldimethylammoniumchlorid, Ditalgdimethylammoniumchlorid oder Dihexadecylammoniumchlorid.

Beispiele für Verbindungen der Formel (V) sind Methyl-N-(2-hydroxyethyl)-N,N-di(talgacyloxyethyl)ammoniummethosulfat, Bis-(palmitoyl)-ethyl-hydroxyethyl-methylammoniummethosulfat oder Methyl-N,N-bis(acyloxyethyl)-N-(2-hydroxyethyl)ammoniummethosulfat.

Um die Benetzbarkeit des textilen Trägermaterials zu verbessern und somit auch die antimikrobielle Wirksamkeit

zu verbessern, kann die Zubereitung insbesondere auch ein Hydrophilierungsmittel umfassen. Beispiele für derartige Hydrophilierungsmittel sind ein- oder mehrwertigen Alkohole, Alkanolamine oder Glycolether, sofern sie mit Wasser mischbar sind. Vorzugsweise werden die Hydrophilierungsmittel ausgewählt Ethylenglykolmethylether, Ethylenglykolethylether, Ethylenglykolpropylether, Ethylenglykolmono-n-butylether, Diethylenglykol-methylether, Diethylenglykolethylether, Propylenglykolmethyl-, -ethyl- oder - propyl-ether, Dipropylenglykolmonomethyl-, oder - ethylether, Diisopropylenglykolmonomethyl-, oder -ethylether, Methoxy-, Ethoxy- oder Butoxytriglykol, 1-Butoxyethoxy-2-propanol, 3-Methyl-3-methoxybutanol, Propylen-glykol-t-butylether, Alkoholen, insbesondere C1-C4-Alkanole, Glykole, Polyethylenglykole, vorzugsweise mit einem Molekulargewicht zwischen 100 und 100000, insbesondere zwischen 200 und 10000, und Polyolen, wie Sorbitol und Mannitol, sowie bei Raumtemperatur flüssiges oder feste Polyethylenglykol, Carbonsäureestern, Polyvinylalkoholen, Ethylenoxid-Propylenoxid-Blockcopolymeren sowie beliebigen Gemischen der voranstehenden.

Gemäß der vorliegenden Erfindung ist der medizinische oder medizintechnische Artikel, eine Binde, eine Polsterbinde, eine Kompressionsbinde, eine Gesichtsmaske, eine Operationsabdeckung, eine Patientenabdeckung oder ein Operationsmantel.

Unter einem Gipsverband befindet sich üblicherweise eine Polsterbinde, die den Tragekomfort des Verbandes erhöhen soll. Mit zunehmender Tragedauer des Gipsverbandes jedoch entsteht unter der Polsterbinde durch die auf der Haut des Patienten befindlichen Keime und Hautschuppen ein für den Patienten unkomfortables Milieu, das unter Umständen zu starkem Juckreiz aber auch zu Geruchsbildung führt. Um den Tragekomfort zu erhöhen wird nun vorgeschlagen, diese Binde mit einer Zubereitung auszustatten, die auf einer ersten Seite der Binde aufgebracht ist, und wobei die Zubereitung Polyhexamethylenguanidin als antimikrobielles Mittel umfasst. Diese antimikrobielle Binde kann im einfachsten Fall durch Tauchen oder Besprühen einer üblichen Polsterbinde mit einer nichtalkoholischen Polyhexamethylenguanidin-Lösung bereitgestellt werden. Nach dem Verdampfen des Lösungsmittels wird somit eine antimikrobielle Polsterbinde erhalten, die aufgrund der Wirkweise von Polyhexamethylenguanidin sehr lange antimikrobiell wirkt und somit auftretende Keime wirkungsvoll abtötet, wodurch keine Geruchsbildung oder Juckreiz entsteht.

Spinnvliese werden im medizinischen Bereich unter anderem als Keimbarriere in Bekleidungen für den Arzt im Operationssaal als so genannter Operationsmantel (OP-Mäntel), oder zur Abdeckung von Patienten während einer Operation als so genannte Patientenabdeckungen eingesetzt.

Wie bereits aufgeführt ist ein Gegenstand der Erfindung ein Operationsmantel, der von einem behandelnden Arzt während einer Operation getragen wird. Ein Standart-Operationsmantel hat eine nach der Norm EN 13795 definierte Dichtigkeit gegenüber Flüssigkeiten. Da es jedoch während der Operationen unter Umständen zum Kontakt mit größeren Mengen an Flüssigkeiten kommen kann, reicht eine gemäß dieser Norm eingestellte Dichtigkeit oftmals nicht aus, um ein Infektionsrisiko vollständig ausschließen zu können. Durch eine mögliche Leckage wird einerseits das Risiko der Keimübertragung vom innen nach außen, d.h. vom Arzt zum Patienten aber auch umgekehrt vom Patienten zum Arzt erhöht. Durch die antimikrobielle Ausrüstung mit einer Polyhexamethylenguanidiniumchlorid umfassenden Zusammensetzung kann dieses Risiko weitgehenst minimiert werden.

Der erfindungsgemäße medizinische oder medizintechnische Artikel, nämlich eine Binde, eine Polsterbinde, eine Kompressionsbinde, eine Gesichtsmaske, eine Operationsabdeckung, eine Patientenabdeckung oder ein Operationsmantel weist eine erste oberflächenbildende Seite auf, die durch die erste oberflächenbildende Seite des Trägermaterials gebildet wird. Diese erste oberflächenbildende Seite des Artikels kann im anwendungsgerechten Zustand zum Patienten oder zum Anwender gerichtet sein. Es kann jedoch auch vorgesehen sein, dass diese erste oberflächenbildende Seite des Artikels vom Patienten oder vom Anwender wegweist. So ist insbesondere auch vorgesehen, das die erste oberflächenbildende Seite des Trägermaterials eine Außenfläche eines Operationsmantels bildet.

Zur Herstellung eines Operationsmantels kann das Trägermaterial, das von dem Mantel umfasst wird, auf einer oder auf beiden Seiten insbesondere mit einer 0,1 - 0,5 % konzentrierten wässrigen Lösung vollständig oder in bestimmten Bereichen, einmalig oder mehrmalig besprüht oder vollständig in die Lösung getaucht und anschließend getrocknet werden. Dieses Trägermaterial kann beispielsweise das Vorderteil, ein Seitenteile und/ oder ein Ärmel des Mantels bilden. Hierbei ist insbesondere vorgesehen, dass die erste oberflächenbildende Seite des Trägermaterials gleichzeitig eine Außenseite des Mantels bildet. Damit umfasst ein erfindungsgemäßer Artikel insbesondere eine erste Außenseite, die durch die erste oberflächenbildende Seite des Trägermaterials gebildet wird.

Weiter ist auch ein Verfahren zur Herstellung eines medizinischen oder medizintechnischen Artikels umfassend ein textiles Trägermaterial mit einer ersten oberflächenbildenden Seite und einer zweiten oberflächenbildenden Seite beschrieben. Insbesondere kann mit diesem Verfahren ein Artikel der oben beschriebenen Art, wie eine Binde, eine Polsterbinde, eine Kompressionsbinde, eine Gesichtsmaske, eine Operationsabdeckung, eine Patientenabdeckung oder ein Operationsmantel jeweils umfassend ein textiles Trägermaterial mit einer ersten oberflächenbildenden Seite und einer zweiten oberflächenbildenden Seite hergestellt werden. Das Verfahren umfasst die Verfahrensschritte:

a) Lösen von Polyhexamethylenguanidin oder ein Salz hiervon, insbesondere Polyhexamethylenguanidiniumchlorid in einem Lösungsmittel L1 zur Herstellung einer Lösung A,

a') Lösen von weiteren Hilfs- oder Zusatzstoffen

- in dem Lösungsmittel L1 oder einem Lösungsmittel L2 zur Herstellung einer Lösung B oder
- in der Lösung A zur Herstellung einer Lösung C erfolgt und

b) ein Besprühen des Trägermaterials auf der ersten Seite oder Tauchen des Trägermaterials oder Tauchen des Artikels

- mit der/ in die Lösung A und mit der/ in die Lösung B oder
- mit einer/ in eine Lösung D, die die Lösung A und die Lösung B enthält oder
- mit der/ in die Lösung C erfolgt,

c) Trocknen des Trägermaterials oder des Artikels.

Insbesondere werden in diesem Verfahren das Lösungsmittel L1 und/ oder das Lösungsmittel L2 aus der Gruppe bestehend aus Alkoholen, Ketonen, aromatischen Lösungsmitteln, Wasser oder einer Mischung hiervon gewählt.

Weiterhin bevorzugt ist in diesem Verfahren, dass die Lösung A, C oder D eine Konzentration von 0,1 bis 20 Gew.-%, vorzugsweise 0,2 bis 15 Gew.-%, an Polyhexamethylenguanidin oder ein Salz hiervon, insbesondere Polyhexamethylenguanidiniumchlorid enthält.

Insbesondere ist auch vorgesehen, dass die Lösung B, C oder D eine Konzentration von 0,1 bis 20 Gew.-%, vorzugsweise 0,2 bis 15 Gew.-%, an weiteren Hilfs- oder Zusatzstoffen enthält.

Hervorzuheben ist an dieser Stelle, dass die hier aufgeführten Merkmale der alternativen oder bevorzugten Ausgestaltungen der Erfindung nicht auf die einzelnen Alternativen oder Bevorzugungen zu beschränken sind. Im Zusammenhang mit der vorliegenden Erfindung ist es vielmehr der Fall, dass eine Kombination von Ausgestaltungen bzw. eine Kombination jedes Einzelmerkmals einer alternativen Form mit Merkmalen einer anderen alternativen Ausgestaltung ebenso als erfindungsgemäßer Gegenstand zu sehen ist. Ebenso wenig soll die Erfindung durch die nachfolgende Beschreibung der Zeichnungen reduziert verstanden sein.

Nachstehend wird die Erfindung unter Bezugnahme auf Zeichnungen anhand eines Beispiels erläutert. In den Zeichnungen zeigt Figur 1 einen erfindungsgemäßen Operationsmantel.

Der in Figur 1 in Form eines Wickelkittels dargestellte Operationsmantel (7) weist erkennbar einen Hauptteil (1) mit einer Außenseite (11) auf, wobei die Außenseite (11) einen ersten Bereich (12) und einen zweiten Bereich (13) aufweist und der zweite Bereich (13) seinerseits durch einen rechteckförmigen, wässrige Flüssigkeiten absorbierenden Materialabschnitt (14) aus einem binderverfestigten Viskosevlies mit der Bezeichnung MH 1491-49, zu beziehen bei der Fa. Fiberduk AB, Ystad, Schweden, gebildet ist. Der Binderanteil (Weichacrylat) dieses Viskosevlieses beträgt 28%; der Viskosefaseranteil beträgt 72%. Das Flächengewicht des Viskosevlieses beträgt 29 g/m$^2$. Die Feinheit der Viskosefasern des Viskosevlieses beträgt 1,5 dtex. Dieser Materialabschnitt (14) wird vor der Verarbeitung zum Mantel in eine 0,5 Gew.-% wässrige Polyhexamethylenguanidiniumchlorid-Lösung (CAS 57 028-96-3) mit einer Verweilzeit von 1 Minuten getaucht. Anschließend wird die überschüssige Lösung abgetropft und das Material bei 40 °C trocknen gelassen, bevor es weiterverarbeitet werden kann. In dieser Ausführung ist sowohl die später zum Mantel gerichtete Seite als auch die im Gebrauch des Mantels außen liegende antimikrobiell ausgerüstet. Der Operationsmantel (7) kann des Weiteren in Figur 1 nicht dargestellte Wickel- oder Bindebänder oder klebende Verschlussmittel oder andere Verschlusselemente aufweisen, um den Operationsmantel (7) am Körper einer Person zu fixieren. Wie in Figur 2 erkennbar ist, ist der wässrige Flüssigkeiten absorbierende Materialabschnitt (14) über ein Klebemittel (8), nämlich einen Hotmeltklebstoff, auf der Außenseite der Chassislage (2) im vorliegenden Fall unlösbar fixiert. Denkbar und vorteilhaft ist im Falle der Verwendung ultraschallschweißbarer, also insbesondere thermoplastischer Materialien außerdem eine Fixierung mittel Ultraschallschweißen. Der Operationsmantel umfasst außerdem zwei Ärmel (6), die im dargestellten Fall an den Hauptteil (1) angefügt sind. Der Hauptteil (1) umfasst neben dem Vorderteil (4) die beiden linken und rechten Rückenteile (5a, 5b). Die Rückenteile (5a, 5b) sind in der dargestellten Ausführungsform entlang je einer Ultraschallschweißnaht (9) an den Vorderteil (4) angefügt. Denkbar und vorteilhaft ist auch, den Hauptteil gleichsam integral aus einem einzigen Zuschnitt zu formen. Die den Hauptteil (1) bildende Chassislage (2) besteht aus einem SMMS-Laminat (Spunbond-Meltblown-Meltblown-Spunbond-Laminat) mit einem Flächengewicht von 35g/m$^2$, das unter der Materialnummer 60385A bei der Fa PGI, Polymer Group - Cuijk, Lange Oijen 16, NL-5433 NG Katwijk, N-Brabant, bezogen werden kann. Das Flächengewicht der Spunbond-Lagen beträgt jeweils 13 g/m$^2$, das der Meltblown-Lagen jeweils 4,5 g/m$^2$. Diese die Chassislagen bildende Material wird vor Verarbeitung zum Mantel in eine 0,5 Gew.-% wässrige Polyhexamethylenguanidiniumchlorid-Lösung (CAS 57 028-96-3) mit einer Verweilzeit von 1 Minuten getaucht. Anschließend wird die überschüssige Lösung abgetropft und das Material bei 40 °C trocknen gelassen, bevor es weiterverarbeitet werden kann. In dieser Ausführung ist sowohl die später im Mantel innen zum Körper des Anwenders liegende Seite des SMMS-Materials auch die im Mantel außen liegende Seite des Mantels antimikrobiell ausgerüstet. Der wässrige

Flüssigkeiten absorbierende Materialabschnitt (14) ist in Längsrichtung RL weiter von einem unteren Ende (15) des Operationsmantels (7) als von einem oberen Ende (16) des Operationsmantels beabstandet. Bezüglich einer zentralen Längsachse L ist der wässrige Flüssigkeiten absorbierende Materialabschnitt (14) hingegen symmetrisch angeordnet. Die in Längsrichtung RL gemessene Abschnittsbreite B des wässrige Flüssigkeiten absorbierenden rechteckigen Materialabschnittes beträgt 30 cm. Die in Querrichtung RQ gemessene Abschnittslänge D des wässrige Flüssigkeiten absorbierenden Materialabschnittes (14) beträgt 80 cm. Somit beträgt die Fläche des wässrige Flüssigkeiten absorbierenden Materialabschnittes (14) 0,24 $m^2$. Die Fläche der Außenseite des aus dem Vorderteil (4) und den beiden Rückenteilen (5a, 5b) bestehenden Hauptteils (1) beträgt inklusive der Fläche des wässrige Flüssigkeiten absorbierenden Materialabschnittes 2,60 $m^2$. Die Fläche des ersten Bereiches (12) beträgt somit 2,36 $m^2$. Die Fläche des ersten Bereiches (12) ist somit um 983 % größer als die des zweiten Bereiches (13).

In Gebrauch kann eine an einer Operation beteiligte Person seine durch wässrige Flüssigkeiten kontaminierten Hände durch Abtupfen oder Abstreifen oder Abwischen an dem wässrige Flüssigkeiten absorbierenden Materialabschnitt (14) reinigen. Durch die antimikrobielle Ausrüstung des Materialabschnitts (14) und der Chassislage (2) ist eine Kontamination des behandelnden Arztes weitgehendst ausgeschlossen. Darüber hinaus kann im Gebrauch des Mantels der so ausgerüstete Operationsmantel Mikroorganismen, die mit der Innenseite und / oder Außenseite desselben in Berührung kommen abtöten, um eine Verschleppung der Mikroorganismen in das sterile Operationsfeld und / oder zur Haut des Trägers des Operationsmantels zu vermeiden.

<u>Prüfung auf antimikrobielle Aktivität</u>

Das Prüfverfahren dient zur quantitativen Bestimmung der Wirkung der antimikrobiellen Ausrüstung bei textilen Materialien. Dazu wird das textile Material mit dem jeweiligen Bakterienstamm, der zuvor in Weichagar gegeben wird, beimpft. Nach definierten Zeiten werden Keimzahlbestimmungen durchgeführt. Das Prüfverfahren wird angelehnt an die ASTM E 2180-01 durchführt.

Für den Versuch werden benötigt:

- Sterile Schere, sterile Pinzette
- Stanzeisen mit Durchmesser 4 cm
- Sterile Flaschen, Petrischalen, Reagenzgläser
- Brutschrank (37°C ± 2°C)
- Ringerlösung mit Enthemmern (0,3% Lecithin, 0,1 % Histidin, 1% Tween). Die Ringerlösung wird durch Auflösen von 1 Ringer-Tablette (erhältlich bei der Fa. Merck unter der Materialnummer 1.15525.0001) in 500 ml destilliertem Wasser hergestellt. Eine Ringertablette enthält 0,00525 g Ammoniumchlorid, 0,005 g Natriumhydrogencarbonat, 0,04 g Calciumchlorid - 2 Hydrat, 0,00525 g Kaliumchlorid und 1,125 g Natriumchlorid.
- CASO-Agar = Caseinpepton-Sojamehlpepton-Agar (erhältlich bei der Fa. Merck unter der Materialnummer 1.05458.0500)
- CASO-Bouillon (erhältlich bei der Fa. Merck unter der Materialnummer 1.05459.0500)
- 0,85%-ige Kochsalzlösung
- Weichagar (hergestellt aus Mischung von 0,85 g Kochsalz, 0,3 g Agar- Agar (erhältlich bei Fa. Merck unter der Materialnummer 1.01614.1000) und 100 ml destilliertes Wasser)
- Bakterienstämme: Staphylococcus aureus ATCC 6538 (DSM 346), Klebsiella pneumoniae ATCC 4352 (DSM 789)

Vorbereitung:

Vom textilen Material werden Prüfstücke mit einem Durchmesser von 4 cm ausgestanzt bzw. geschnitten. Die Anzahl der Prüfstücke wird so gewählt, dass mindestens eine Dreifachbestimmung zu den definierten Zeiten nach der Beimpfung möglich ist. Unter Prüfstücke werden dabei die mit antimikrobiellem Wirkstoff ausgerüsteten Probenmaterialstücke sowie die Kontrollmaterialstücke ohne antimikrobielle Wirkstoffausrüstung verstanden.

Die Prüfstücke werden unter den für das Prüfstück geeigneten Bedingungen sterilisiert. Für den Versuch wird der jeweilige Bakterienstamm mit Kolonien von 1-5 x $10^a$ KBE/ml (KBE = Kolonie bildende Einheiten) eingesetzt.

Versuchsdurchführung:

Bei dem gesamten Prüfverfahren wird die eingesetzte Keimzahl zusätzlich überprüft, d.h. sämtliche Prüfverfahrensschritte werden ohne Einbringen eines Prüfstücks durchgeführt (Keimzahl-Kontrolle).

Jedes Prüfstück (also Proben - und Kontrollmaterialstück) wird in eine Petrischale gegeben. Mittels eines sterilen Tupfers, welcher mit einer sterilen 85 %-igen Kochsalzlösung getränkt ist, wird jedes Prüfstück durch Abtupfen befeuchtet. Diese Befeuchtung gewährleistet eine gleichmäßige Verteilung des nachfolgend aufzubringenden Weichagars.

1 ml der Bakterienkultur mit 1-5 x $10^8$ KBE/ml wird in 100 ml des auf 45 ± 2°C eingestellten Weichagars gegeben. Damit liegen 1-5 x $10^6$ KBE/ml Bakterienkultur im Weichagar vor. Anschließend wird 1 ml des beimpften Weichagars vorsichtig auf das Prüfstück aufgebracht, derart dass ein dünner Film auf dem gesamten Prüfstück entsteht. Nach Erstarren des Weichagars werden die mit den Prüfstücken beladenen Petrischalen bis zum gewünschten Prüfzeitpunkt zur Keimzahlbestimmung bei 37 ± 2 °C in den Brutschrank gestellt.

Als Prüfzeitpunkte können beispielsweise Zeit 1= 0 h (also sofort, direkt nach Beladung des Prüfstücks mit beimpften Weichagar), Zeit 2= 4 h, Zeit 3 = 24 h oder noch spätere Zeitpunkte gewählt werden.

Zur Keimzahlbestimmung werden die Prüfstücke mittels 100 ml Ringerlösung mit Enthemmer jeweils in eine sterile 250 ml Schraubverschlussflasche gegeben. Zum Ablösen des Weichagars vom Prüfstück wird das Schraubverschlussgefäß für 1 min im Ultraschallbad behandelt und anschließend für 1 min mechanisch geschüttelt. Nachfolgend wird daraus eine Verdünnungsreihe angelegt, indem jeweils 1 ml aus der vorangehenden Lösung in ein steriles Röhrchen mit vorgelegten 9 ml an Ringerlösung mit Enthemmer eingebracht wird. Die Verdünnungsreihen werden derart angelegt, so dass eine spätere Auszählung der herangewachsenen Kolonien möglich ist. Bei Ansätzen mit zu erwartendem höheren Bakterienwachstum, wie den Kontrollmaterialstücken und den Keimzahl-Kontrollen, sind demnach höhere Verdünnungen anzusetzen.

Anschließend wird mit einer sterilen Pipette aus jeder Verdünnungsstufe 1 ml entnommen und in eine sterile Petrischale pipettiert und mit 15 - 20 ml Agarmedium (CASO-Agar) beschichtet. Zur gleichmäßigen Verteilung des Mediums werden die befüllten Petrischalen kreisend über die Arbeitsfläche bewegt. Nach Erstarren des Agars werden die Petrischalen mit dem Deckel nach unten in den Brutschrank gestellt und 18 - 24 Stunden bei 37 ± 2°C im Brutschrank bebrütet.

Die Auswertung der Keimzahlen erfolgt dann mittels Abzählen der gewachsenen Keimkolonien. Bei stärkerem Kolonienwachstum sind die Platten mit Kolonien zwischen 30 und 300 zur Auswertung heranzuziehen und möglichst die Zählwerte zweier Verdünnungsstufen zu berücksichtigen.

Die mittlere geschätzte Kolonienzahl ermittelt sich wie folgt:

$$x_m = \frac{\text{Summe Koloniezählwert}}{\text{Summe Verdünnungsstufen}}$$

$$\text{bspw.}\quad x_m = \frac{303 + 290 + 32 + 28}{10^{-4} + 10^{-4} + 10^{-5} + 10^{-5}} = \frac{653}{2{,}2 \times 10^{-4}} = 2{,}97 \times 10^6$$

Die ermittelten Werte werden dann in der Einheit KBE/g oder KBE/ml angegeben.

<u>Beispiel: Prüfung der antimikrobiellen Aktivität von PHMG auf Vliesstoff.</u>

Als Wirkstoff wird eine 3,3 %-ige Lösung von PHMG (Polyhexamethylenguanidiniumchlorid; CAS 57028-96-3) eingesetzt. Diese Lösung kann bei der Firma Oy Soft Protector Ltd., Nupurintie 4, FIN-02761 Espoo, unter dem Handelsnamen Desisoft 20 bezogen werden.

Als textiles Material wird ein SMMMS-Vlies (S= Spunbond, M=Meltblown) aus Polypropylen mit einem Gesamtflächengewicht von 35 g/m$^2$ eingesetzt. Das Vlies kann bei der Fa. PGI bezogen werden.

Zur Ausrüstung mit PHMG wird das textile Material 5 Sekunden in diese Wirkstofflösung eingetaucht. Die überschüssige Lösung wird abgetropft und das textile Material wird trocknen gelassen. Aus diesem nun mit PHMG ausgerüstetem Vlies werden die entsprechenden Probenmaterialstücke wie oben beschrieben ausgestanzt.

Aus dem nicht mit PHMG ausgerüstetem Vlies werden entsprechende Kontrollmaterialstücke ausgestanzt.

Die Prüfstücke werden mittels Ethylenoxid in einem validierten Verfahren (780 mg/l; 180 min, 50 ± 2 °C) sterilisiert.

Dabei werden hierbei bei den mit PHMG-behandelten Probenmaterialstücke zwei Ansätze durchgeführt. Ansatz 1: Das Probenmaterialstück wird zuerst mit PHMG ausgerüstet und dann sterilisiert. Ansatz 2: Das Probenmateriastück wird zuerst sterilisiert und dann mit PHMG ausgerüstet. Die Versuchsdurchführung erfolgt dann wie oben beschrieben.

Die Keimzahlbestimmungen erfolgen nach den Inkubationszeiten Zeit 1 =0 h, Zeit 2 = 4 h und Zeit 3 = 24 h.

Die Ergebnisse in Tabelle 1 und Tabelle 2 zeigen die antimikrobielle Wirkung von PHMG auf dem textilen Material Vliesstoff.

Tabelle 1: Antimikrobielle Wirksamkeit bei Versuchskeim Staphylococcus aureus DSM 346

| | Nach 0 h (sofort) [KBE/g] | Nach 4 h [KBE/g] | Nach 24 h [KBE/g] |
|---|---|---|---|
| SMMMS - Vlies ausgerüstet mit PHMG 3,3% + sterilisiert (Probenmaterial) | 1,00E+00 | 1,00E+00 | 1,00E+00 |
| SMMMS - Vlies sterilisiert + ausgerüstet mit PHMG 3,3% (Probenmaterial) | 1,00E+00 | 1,00E+00 | 1,00E+00 |
| SMMMS - Vlies sterilisiert (Kontrollmaterial) | 8,10E+04 | 8,66E+04 | 3,05E+06 |
| Keimzahl-Kontrolle | 9,06E+04 | 5,96E+05 | 4,61 E+06 |

Tabelle 2: Antimikrobielle Wirksamkeit bei Versuchskeim Klebsiella pneumoniae DSM 789

| | Nach 0 h (sofort) [KBE/g] | Nach 4 h [KBE/g] | Nach 24 h [KBE/g] |
|---|---|---|---|
| SMMMS - Vlies ausgerüstet mit PHMG 3,3% + sterilisiert (Probenmaterial) | 1,48E+02 | 1,00E+00 | 1,00E+00 |
| SMMMS - Vlies sterilisiert + ausgerüstet mit PHMG 3,3% (Probenmaterial) | 6,50E+01 | 1,00E+00 | 1,00E+00 |
| SMMMS - Vlies sterilisiert (Kontrollmaterial) | 6,75E+04 | 6,40E+04 | 3,90E+05 |
| Keimzahl-Kontrolle | 7,36E+04 | 3,75E+05 | 4.45E+06 |

**Patentansprüche**

1. Medizinischer oder medizintechnischer Artikel umfassend eine antimikrobielle Zubereitung und ein textiles Trägermaterial mit einer ersten oberflächenbildenden Seite und einer zweiten oberflächenbildenden Seite, wobei die Zubereitung auf der ersten Seite des Trägermaterials aufgebracht ist, **dadurch gekennzeichnet, dass** die Zubereitung Polyhexamethylenguanidin oder ein Salz hiervon als antimikrobielles Mittel und als weitere Hilfs- oder Zusatzstoffe ein Hautpflegemittel, ein Tensid, ein Hydrophobisierungsmittel, ein Hydrophilisierungsmittel und/ oder ein Avivagemittel umfasst und wobei der Artikel eine Binde, eine Polsterbinde, eine Kompressionsbinde, eine Gesichtsmaske, eine Operationsabdeckung, eine Patientenabdeckung oder ein Operationsmantel ist.

2. Medizinischer oder medizintechnischer Artikel gemäß Anspruch 1, **dadurch gekennzeichnet, dass** das textile Trägermaterial ein Faservlies, ein Gewirk, ein Gewebe oder ein Gestrick ist.

3. Medizinischer oder medizintechnischer Artikel gemäß Anspruch 2, **dadurch gekennzeichnet, dass** das Faservlies ein Stapelfaservlies, ein Spinnvlies oder ein Meltblownvlies ist.

4. Medizinischer oder medizintechnischer Artikel gemäß mindestens einem der vorgenannten Ansprüche, **dadurch gekennzeichnet, dass** der Gehalt an Polyhexamethylenguanidin oder dessen Salz bezogen auf die Oberfläche des Artikels 0,0001 bis 100 mg/ $cm^2$ beträgt.

5. Medizinischer oder medizintechnischer Artikel gemäß mindestens einem der vorgenannten Ansprüche, **dadurch gekennzeichnet, dass** der Artikel mindestens eine Abstandsschicht mit einer ersten oberflächenbildenden Seite und einer zweiten oberflächenbildenden Seite aufweist, wobei die erste Seite der Abstandsschicht der ersten Seite des Trägermaterials benachbart ist.

6. Medizinischer oder medizintechnischer Artikel gemäß mindestens einem der vorgenannten Ansprüche, **dadurch gekennzeichnet, dass** die erste oberflächenbildende Seite des Trägermaterials eine Außenseite des Artikels bildet.

**Claims**

1. Medicinal or medical article comprising an antimicrobial preparation and a textile backing material having a first

surface-forming side and a second surface-forming side, wherein the preparation is on the first side of the backing material, **characterized in that** the preparation comprises polyhexamethyleneguanidine or a salt thereof as antimicrobial agent and, as further auxiliary or added substances, a skin care agent, a surfactant, a hydrophobicizing agent, a hydrophilicizing agent and/or a hand-modifying agent and wherein the article is a bandage, a cushioning bandage, a compression bandage, a face mask, a surgery drape, a patient cover or a surgical gown.

2. Medicinal or medical article according to Claim 1, **characterized in that** the textile backing material is a fibre web, a loop-formingly knitted fabric, a woven fabric or a loop-drawingly knitted fabric.

3. Medicinal or medical article according to Claim 2, **characterized in that** the fibre web is a staple fibre web, a spunbonded or a meltblown web.

4. Medicinal or medical article according to at least one aforementioned claim, **characterized in that** the content of polyhexamethyleneguanidine or its salt is from 0.0001 to 100 $mg/cm^2$, based on the surface area of the article.

5. Medicinal or medical article according to at least one aforementioned claim, **characterized in that** the article has at least one spacer layer having a first surface-forming side and a second surface-forming side, wherein the first side of the spacer layer is adjacent to the first side of the backing material.

6. Medicinal or medical article according to at least one aforementioned claim, **characterized in that** the first surface-forming side of the backing material forms an exterior side of the article.

**Revendications**

1. Article médical ou de technologie médicale, comprenant une préparation antimicrobienne et une matière textile de support comportant une première face constituant la surface et une deuxième face constituant la surface, la préparation étant appliquée sur la première face de la matière de support, **caractérisé en ce que** la préparation comprend de la polyhexaméthylèneguanidine ou un sel de celle-ci en tant qu'agent antimicrobien et comme autres additifs ou adjuvants un agent pour le soin de la peau, un tensioactif, un agent d'hydrophobisation, un agent d'hydrophilisation et/ou un agent d'avivage et l'article étant une bande élastique, une bande élastique matelassée, une bande élastique de compression, un masque filtrant, un champ opératoire, une chemise fendue ou une blouse d'opération.

2. Article médical ou de technologie médicale selon la revendication 1, **caractérisé en ce que** la matière textile de support est un non-tissé de fibres, un tricot, un tissu ou un tissu à mailles.

3. Article médical ou de technologie médicale selon la revendication 2, **caractérisé en ce que** le non-tissé de fibres est un non-tissé de fibres discontinues, un non-tissé en voie fondue ou un non-tissé meltblown.

4. Article médical ou de technologie médicale selon au moins l'une quelconque des revendications précédentes, **caractérisé en ce que** la teneur en polyhexaméthylèneguanidine ou en un sel de celle-ci vaut de 0,0001 à 100 $mg/cm^2$ par rapport à la surface de l'article.

5. Article médical ou de technologie médicale selon au moins l'une quelconque des revendications précédentes, **caractérisé en ce que** l'article comporte au moins une couche d'écartement comportant une première face constituant la surface et une deuxième face constituant la surface, la première face de la couche d'écartement étant contiguë à la première face de la matière de support.

6. Article médical ou de technologie médicale selon au moins l'une quelconque des revendications précédentes, **caractérisé en ce que** la première face constituant la surface de la matière de support constitue la face externe de l'article.

Figur 1

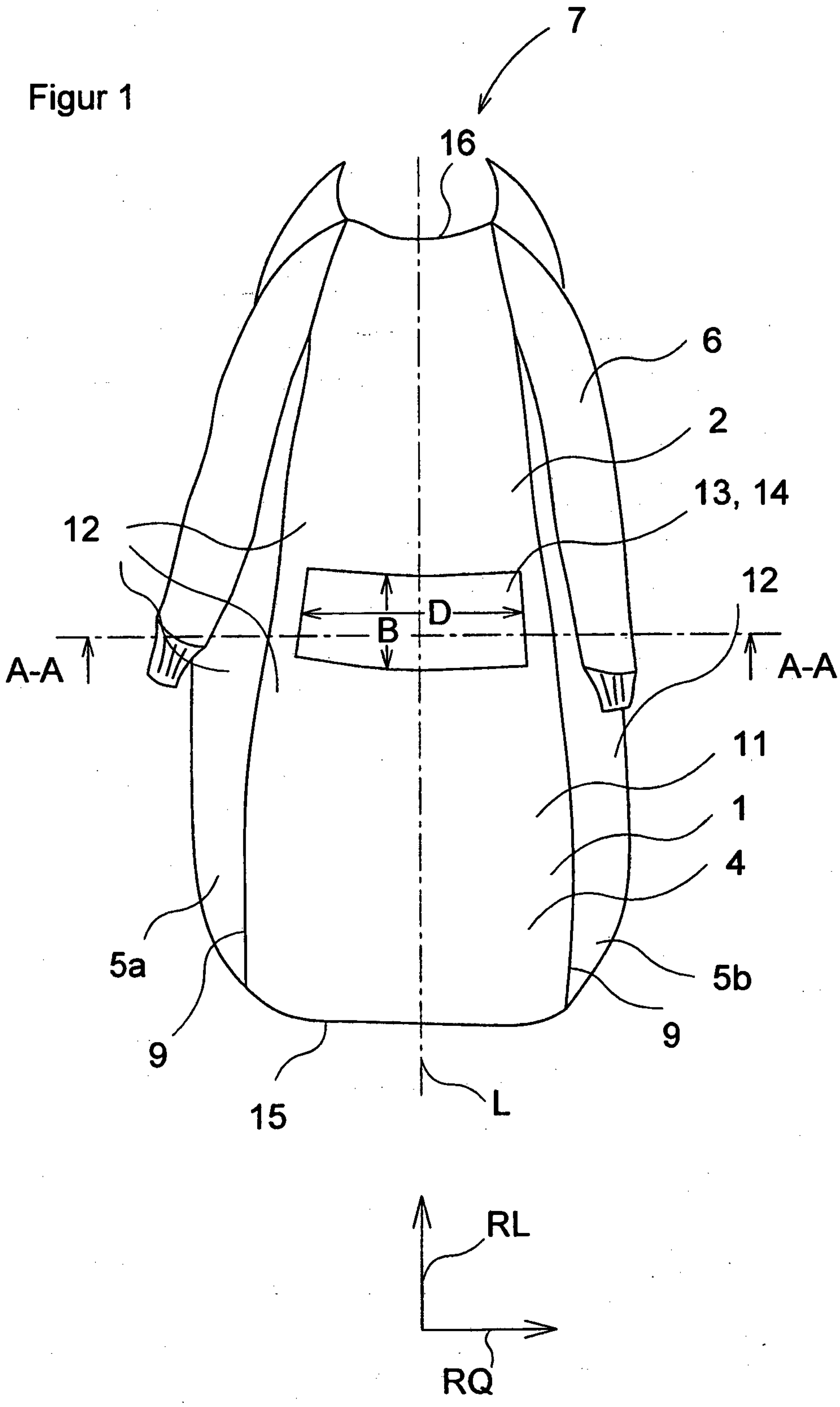

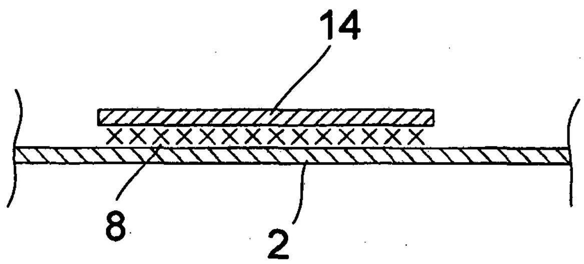


Figur 2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 102004011293 A1